# EUROPEAN PATENT APPLICATION

(11) **EP 4 678 400 A2**
(43) Date of publication of application: **14.01.2026**
(21) Application number: 25210296.7
(22) Date of filing: 10.04.2020
(51) Int. Cl.: B33Y 70/00

(54) **BIOMIMETIC SCAFFOLD FOR PERIPHERAL NERVE INJURIES**

(30) Priority: 11.04.2019 US 201962832681 P
(62) Divisional of application: 20788207.7
(71) Applicant: The Regents of the University of California, Oakland, CA 94607-5200 (US)
(72) Inventor: Tuszynski, Mark H., La Jolla, California, 92093 (US); Koffler, Yacov, La Jolla, California, 92093 (US); Lazarovits, Isac, La Jolla, California, 92093 (US)
(74) Representative: Potter Clarkson

(57) **Abstract**

The disclosure relates to a nerve repair scaffold comprising: a sheath having a proximal end and a distal end, the sheath housing a plurality of microchannels traversing the sheath from the proximal end to the distal end, wherein the microchannels are configured to allow growth of nerve tissue; and a first overhang at the proximal end and a second overhang at the distal end.

## Description

### CROSS-REFERENCE TO RELATED APPLICATIONS

The present application claims the benefit of U.S. Provisional Patent Application 62/832,681 filed April 11, 2019, the entire contents of which is incorporated by reference herein.

### FIELD

The present disclosure relates to biomimetic scaffolds incorporating porous microchannels to promote neural tissue growth and methods for making such scaffolds.

### BACKGROUND

Although the peripheral nervous system (PNS) has a greater capacity for regeneration than the central nervous system (CNS), functional regeneration after injury is largely incomplete if injured axons become misaligned or lose contact with innervated tissues. Major functional deficits result, including insufficient re-innervation of target tissues and painful neuroma formation.

Factors that influence PNS regeneration include the nature and the level of the damage itself, the period of denervation, the type and diameter of the damaged nerve fibers, and age. Proximal nerve injuries or complete transection with a large gap of the nerve generally have poorer outcomes with minimal clinically meaningful motor and sensory recovery. Several reasons contributing to suboptimal recovery have been identified and include: 1) deficiencies in rate of axonal regrowth; 2) compromise to an otherwise permissive environment for axonal elongation; 3) changes in the target tissue or path to reach the target tissue; 4) excessive and chronic neuroinflammation; and 5) Schwann cell atrophy and dysfunction.

Currently, the standard in clinical practice for surgical repair of peripheral nerve interface (PNI), in which there is a large gap in the peripheral nerve, involves placement of autologous nerve grafts. Disadvantages of autografts include: 1) donor site morbidity; 2) limited supply of donor grafts; and 3) increased time and complexity of surgery.

Experimental development of scaffolds to support peripheral nerve repair have resulted in commercially available nerve guides, but these single channel nerve guides provide only single large diameter tubes that result in misalignment of regenerating axons with their proper targets. Upon implantation in a transected rat sciatic nerve model, such an open tube single channel nerve guide scaffold results in many axons undesirably losing linear orientation along a proximal end, only 200 µm after they enter the scaffold, prior to reaching the other distal end. As a result, axons are less dense and of those that reach the distal end, some still lose orientation even as they exit into the distal nerve. This misguidance of axons can cause pain due to neuroma.

Recently, cellular approaches including development of conduits filled with Schwann cells have shown some success because Schwann cells naturally support axonal regeneration by guiding and supporting axon growth, but these cells have not been translated for human peripheral nerve injury.

Moreover, there are no effective therapies for promoting regeneration after either acute or chronic spinal cord injuries (SCI) in humans. Various experimental approaches promote axonal regeneration in SCI animal models, including cell grafting to sites of injury to support axonal attachment and elongation. Grafted cells include astrocytes, Schwann cells, marrow stromal cells, or stem cells. However, a drawback of cellular implants is a lack of three-dimensional (3D) organization, resulting in random direction of axon growth; most axons do not regenerate beyond the injury site into host tissue, and hence functional recovery is extremely modest if present at all.

Thus, there remains a need to identify strategies and technologies for enhancing the extent, rate, guidance, targeting, and lesion-distance over which neural tissue (e.g., axons) can regenerate.

### SUMMARY

Disclosed herein are nerve repair scaffolds comprising: a sheath having a proximal end and a distal end, the sheath housing a plurality of microchannels traversing the sheath from the proximal end to the distal end, wherein the microchannels are configured to allow growth of nerve tissue; and a first overhang at the proximal end and a second overhang at the distal end, wherein the first overhang and the second overhang are configured for suturing of nerve tissue.

Also disclosed herein are nerve repair scaffolds comprising: a sheath having a proximal end and a distal end, the sheath housing a plurality of microchannels traversing the sheath from the proximal end to the distal end, wherein the microchannels are configured to allow growth of nerve tissue, and wherein at least one of the microchannel walls comprises a biofunctional agent incorporated into the microchannel wall.

Also disclosed herein are nerve repair scaffolds comprising: a sheath having a proximal end and a distal end, the sheath housing a plurality of microchannels traversing the sheath from the proximal end to the distal end, wherein the microchannels are configured to allow growth of nerve tissue; a first overhang at the proximal end and a second overhang at the distal end, wherein the first overhang and the second overhang are configured for suturing of nerve tissue; wherein the scaffold further comprises a biofunctional agent; wherein each of the microchannels have an open dimeter of about 200 µm to about 350 µm; and wherein the scaffold is prepared from about 15% to about 25% poly(ethylene glycol) diacrylate and about 1-7% methacrylated gelatin.

Also disclosed herein are methods of restoring nerve function comprising implanting the nerve repair scaffold disclosed herein into a nerve injury site in a subject in need thereof, thereby allowing restoration of nerve function across the injury site.

### BRIEF DESCRIPTION OF THE DRAWINGS

FIG. 1 depicts a perspective view of a scaffold as disclosed herein featuring close-packed hexagonal channels.
FIG. 2 depicts a cross-sectional view of a scaffold as disclosed herein featuring close-packed hexagonal channels.
FIG. 3 depicts a side view of a scaffold as disclosed herein featuring close-packed hexagonal channels.
FIG. 4 depicts a perspective view of an alternative embodiment of a scaffold disclosed herein featuring circular channels.
FIG. 5 depicts a cross-sectional view of an alternative embodiment of a scaffold disclosed herein featuring circular channels.
FIG. 6 depicts a side view of an alternative embodiment of a scaffold disclosed herein featuring circular channels.
FIG. 7 depicts a cross section image showing closely packed hexagonal microchannels in a biomimetic scaffold.
FIG. 8 depicts a scaffold holding a suture.
FIG. 9A-B depicts a scaffold disclosed herein implanted into a 1 cm transected rat sciatic nerve, four weeks post implant. FIG. 9A depicts regenerating axons which are misaligned and rarely reach the distal end of an injured nerve in a control animal without an implanted scaffold. FIG. 9B depicts regenerating axons guided by the disclosed multichannel scaffold to reach the distal end of the injured nerve.
FIG. 10 depicts the improved connectivity of spinal cord motor neurons to peripheral muscles in animals using the disclosed multichannel scaffold.
FIG. 11 depicts the improved function of neurons in animals implanted with the disclosed multichannel scaffold evidenced by a significant increase in muscle weight relatively to open tube implant.
FIG. 12 depicts an intact sciatic nerve and sural nerve in a rat specimen.
FIG. 13 depicts a multichannel scaffold provided herein installed across a sciatic nerve gap.

### DETAILED DESCRIPTION

In the natural peripheral nerve, axons are bundled together in fascicles. A gap injury in the peripheral nerve destroys this structure. In cases of an injury to a peripheral nerve (such as the median nerve), where there is a gap that cannot be closed by direct suturing of the two nerve stumps, a bridge is needed. The disclosed multichannel biomimetic scaffolds can both bridge a nerve injury and provide guidance for growth of axons across an injury site. The microchannels in the disclosed scaffold organize the axons and preserve the fidelity of regeneration. Thus, the scaffold keeps them in the same coordinates in space and guides them along the same pathway to the other side of the injury site.

In some embodiments, a scaffold of the desired length is 3D-printed and then placed in the injury site. The proximal and distal nerve stumps are then inserted into the overhangs of the scaffold where they are aligned to the microchannel scaffold. The nerve epineurium is then sutured to the overhang or sheath, thus fixing the scaffold in place. Regenerating axons from the proximal side enter the scaffold and are guided through the injury site to the distal nerve stump. In some embodiments, the channels of the scaffold are filled with Schwann cells to further support axonal regeneration. In some embodiments, neurotrophic factors (such as bone derived neurotrophic factor [BDNF] or nerve growth factor [NGF]) or drug delivery particles can be encapsulated inside the scaffold walls for a controlled release.

Following severe trauma, the nervous system does not spontaneously regenerate, requiring intervention to restore function. There is a need to develop materials that enable the fabrication and implementation of improved and more effective nerve guidance scaffolds. In various aspects, the present disclosure contemplates an improved and more effective tissue scaffold for promoting neural tissue growth and proliferation in a subject. The subject may be an animal with a complex nerve system, such as a mammal, like a human, primate, or companion animal. The tissue scaffolds according to the present disclosure may thus be devices implanted in such a subject.

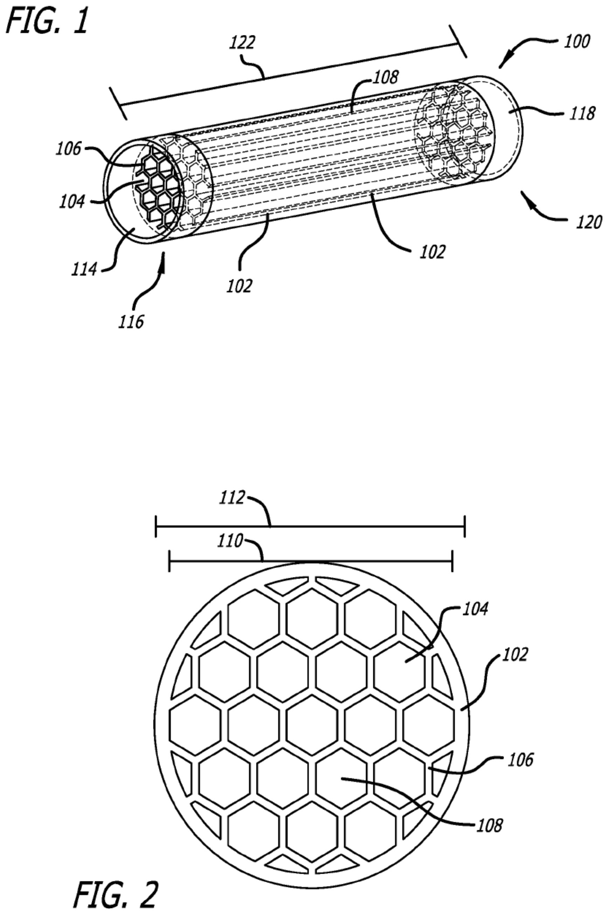

FIG. 1-3 depict one embodiment of disclosed biomimetic scaffold as described herein, scaffold 100. Scaffold 100 includes a sheath (or outer wall) 102. Inside the sheath 102, a plurality of hexagonal microchannels 104 are disposed. Each hexagonal microchannel 104 can include a channel wall 106 and an open lumen 108. In some embodiments, the hexagonal microchannels are more uniform with thinner walls leaving more open space for axons to regenerate through, compared to round or circular microchannels. One particular embodiment shows seven more or less complete hexagons in a given overall scaffold inner diameter 110. Other embodiments can have as many as 200 or more complete hexagons within an inner diameter.

Scaffold 100 can have an outer diameter 112 and an inner diameter 110. Scaffold 100 can further include a first overhang 114 on proximal end 116 and a second overhand 118 on distal end 120. First overhang 114 and/or second overhang 118 can be used for suturing to nerve tissue. Including first overhang 114 and second overhang 118, scaffold 100 can have an overall length 120 including the overhangs.

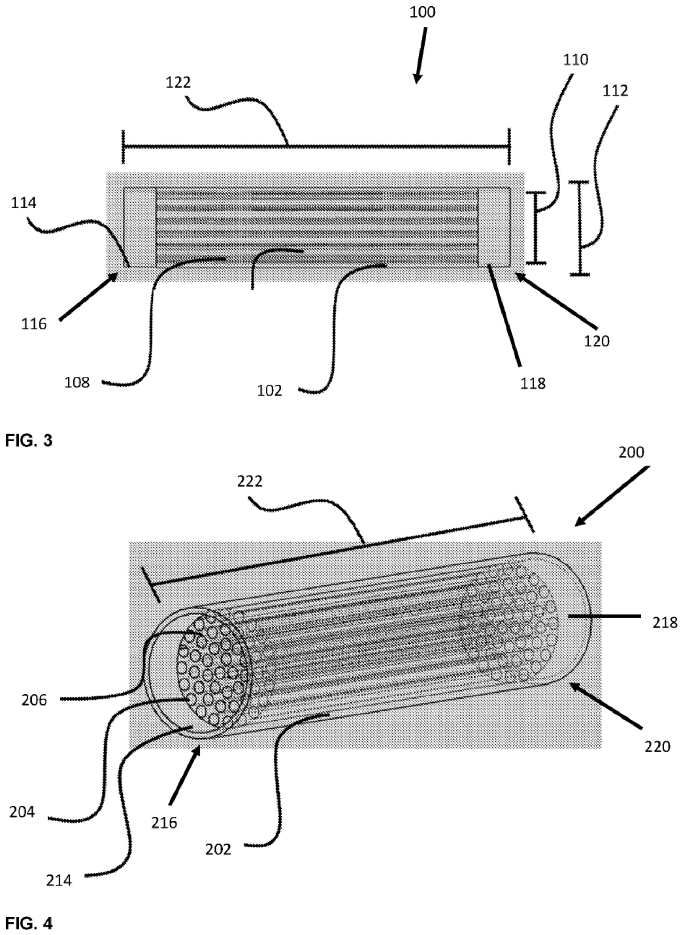

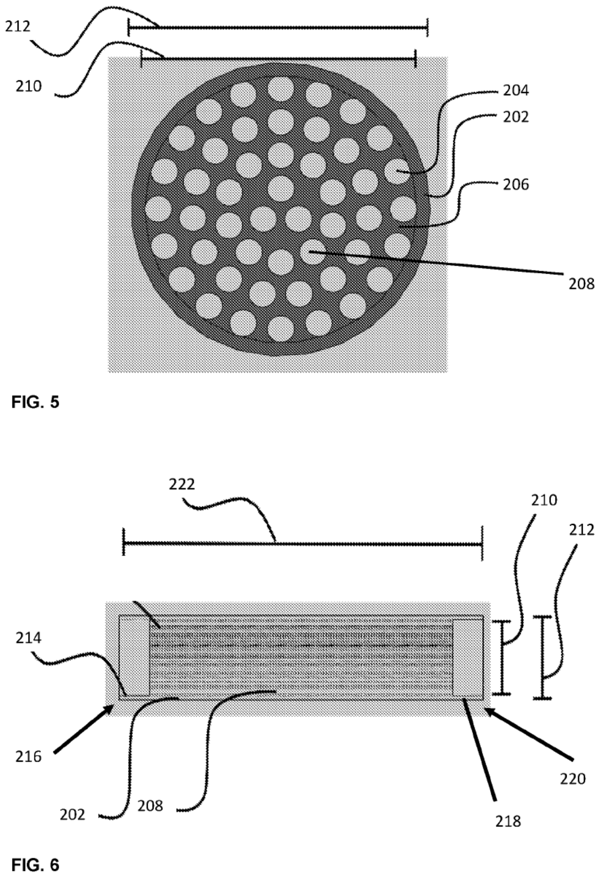

FIG. 4-6 depict another embodiment of a biomimetic scaffold as described herein, scaffold 200. Scaffold 200 includes a sheath (or outer wall) 202. Inside sheath 202, a plurality of circular microchannels 204 are disposed. Each circular microchannel can include a channel wall 206 and an open lumen 208. In some embodiments, the circular microchannels are uniform with thinner walls leaving open space for axons to regenerate through. One particular embodiment shows fifty more or less complete microchannels in a given overall scaffold inner diameter 210. Other embodiments can have as many as 200 or more complete microchannels within an inner diameter.

Scaffold 200 can have an outer diameter 212 that compliments inner diameter 210. Scaffold 200 can further include a first overhang 214 on proximal end 216 and a second overhang 218 on distal end 220. First overhang 214 and/or second overhang 218 can be used for suturing to nerve tissue. Including first overhang 214 and second overhang 218, scaffold 200 can have an overall length 222 including the overhangs.

By "microchannel" it is meant that the structure defines an evident longitudinal axis and has an open lumen or hollow core. In some embodiments, the microchannels are hexagonal or round in shape. In some embodiments, the microchannels can have other generally round or circular shapes or other rectilinear shapes such as, but not limited to hexagonal, round, triangular, rectangular, square, pentagonal, heptagonal, octagonal, nonagonal, decagonal, elliptical, trapezoidal, or other shapes. In some embodiments, the microchannels are substantially round. In some embodiments, a scaffold comprises a mixture of microchannel shapes, for example a mixture of pentagonal and hexagonal microchannels. Microchannels having such an evident longitudinal axis include an elongated axial dimension, which is longer than the other dimensions (e.g., diameter or width) of the channel. Thus, the elongated microchannels are linear.

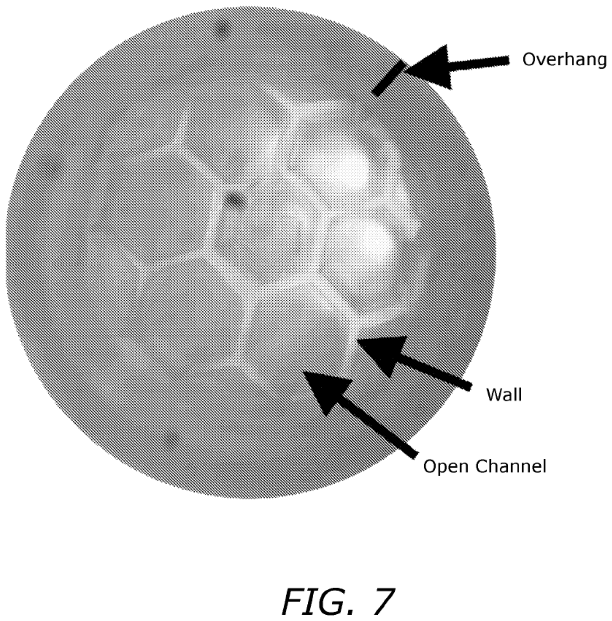

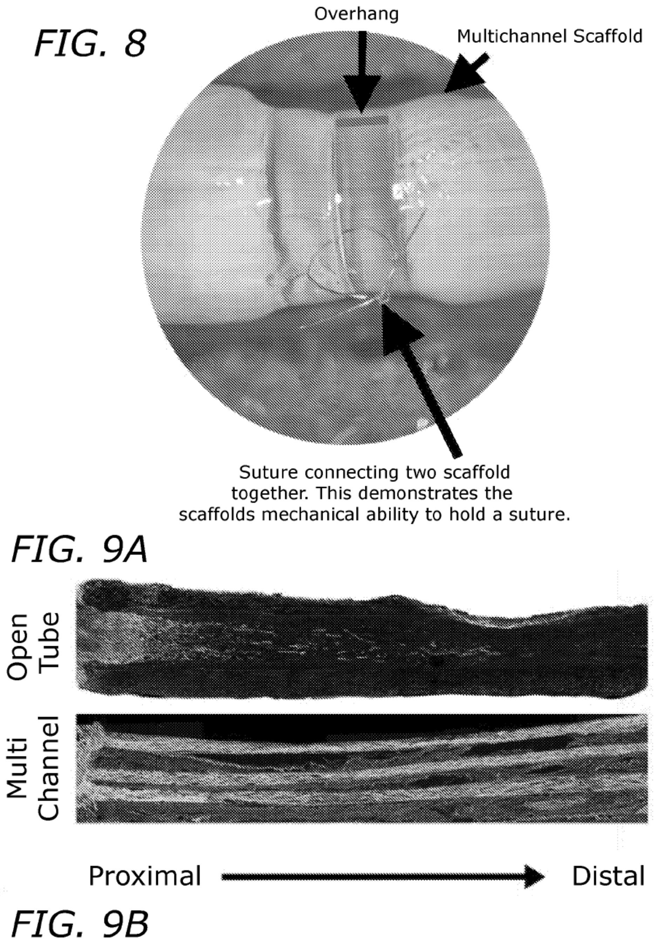

FIGs. 7 and 8 illustrate general features of scaffolds as described herein including overhangs, microchannels, and walls.

In some embodiments, the proximal and distal ends of the scaffold feature "overhangs" as illustrated in the figures and previously described which comprise the sheath overhanging the microchannels so as to provide a substrate to suture to the nerve. In other embodiments, the overhang may be omitted. The overhang can have a length from about 0.1 mm to about 3 mm to allow a medical professional sufficient material to suture the nerve. In some embodiments, the thickness of the overhang is about 1 mm to about 3 mm. In some embodiments, the thickness of the overhang is about 0.1 mm to about 3 mm.

The present disclosure thus contemplates a scaffold comprising a plurality of microchannels respectively defining a longitudinal major axis. In accordance with certain variations of the present disclosure, a "microchannel" preferably has at least one spatial dimension that is less than about 1,000 µm. In certain aspects, each microchannel has an inner diameter of greater than or equal to about 10 µm to less than or equal to about 1,000 µm, optionally greater than or equal to about 10 µm to less than or equal to about 500 µm, optionally greater than or equal to about 50 µm to less than or equal to about 450 µm, optionally greater than or equal to about 50 µm to less than or equal to about 300 µm.

In some embodiments, the microchannels have an open diameter of about 200 µm to about 500 µm, for example about 300 µm. In some embodiments, the microchannels have an open diameter of about 200 µm, about 300 µm, about 400 µm, or about 500 µm. In some embodiments, the microchannels have an open diameter from about 150 µm to about 250 µm. In some embodiments, the microchannels have an open diameter from about 170 µm to about 230 µm. In some embodiments, the microchannels have an open diameter from about 180 µm to about 220 µm. In some embodiments, the microchannels have an open diameter from about 190 µm to about 210 µm. In some embodiments, the microchannels have an open diameter from about 200 µm to about 350 µm. The open diameter refers to the diameter of the lumen of the microchannel. In some embodiments, the wall thickness is about 10 µm to about 50 µm. In some embodiments, the microchannel wall thickness is about to 10 µm to about 60 µm. In some embodiments, the microchannel wall thickness is less than 60 µm, less than 50 µm, less than 40 µm, less than 30 µm, or less than 20 µm. In some embodiments, the microchannel wall thickness is from about 10 µm to about 60 µm, from about 10 µm to about 50 µm, from about 10 µm to about 40 µm, from about 10 µm to about 30 µm, or from about 10 µm to about 20 µm.

For example, depending on the application, microchannels in accordance with certain variations of the present disclosure may have a length of greater than or equal to about 500 µm to less than or equal to 30 cm, optionally greater than or equal to about 500 µm to less than or equal to about 10 cm, and in certain variations, optionally greater than or equal to about 500 µm to less than or equal to about 3 cm, by way of non-limiting example. In some embodiments, the scaffold can be from 0.5 mm to 10 cm in length. In some embodiments, the scaffold can be from 5 mm to 10 cm in length. In some embodiments, the scaffold can be up to 15 cm in length. In some embodiments, the length of the scaffold is about 0.5 cm to about 10 cm. In some embodiments, the length of the scaffold is about 0.5 cm to about 1 cm, about 0.5 cm to about 2 cm, about 0.5 cm to about 3 cm, about 0.5 cm to about 5 cm, about 0.5 cm to about 7 cm, about 0.5 cm to about 9 cm, about 0.5 cm to about 10 cm, about 1 cm to about 2 cm, about 1 cm to about 3 cm, about 1 cm to about 5 cm, about 1 cm to about 7 cm, about 1 cm to about 9 cm, about 1 cm to about 10 cm, about 2 cm to about 3 cm, about 2 cm to about 5 cm, about 2 cm to about 7 cm, about 2 cm to about 9 cm, about 2 cm to about 10 cm, about 3 cm to about 5 cm, about 3 cm to about 7 cm, about 3 cm to about 9 cm, about 3 cm to about 10 cm, about 5 cm to about 7 cm, about 5 cm to about 9 cm, about 5 cm to about 10 cm, about 7 cm to about 9 cm, about 7 cm to about 10 cm, or about 9 cm to about 10 cm. In some embodiments, the length of the scaffold is about 0.5 cm, about 1 cm, about 2 cm, about 3 cm, about 5 cm, about 7 cm, about 9 cm, or about 10 cm. In some embodiments, the length of the scaffold is at least about 0.5 cm, about 1 cm, about 2 cm, about 3 cm, about 5 cm, about 7 cm, or about 9 cm. In some embodiments, the length of the scaffold is at most about 1 cm, about 2 cm, about 3 cm, about 5 cm, about 7 cm, about 9 cm, or about 10 cm. In some embodiments, the length of the scaffold is form about 0.1 cm to about 15 cm. In some embodiments, the length of the scaffold is from about 5 cm to about 15 cm. In some embodiments, the length of the scaffold is at least 5 cm.

The outer diameter of the scaffold can be from about 1.5 mm to about 10 mm, or any diameter between 1.5 mm and 10 mm. In some embodiments, the outer diameter of the scaffold is about 1.5 mm to about 10 mm. In some embodiments, the outer diameter of the scaffold is about 1.5 mm to about 2.5 mm, about 1.5 mm to about 3.5 mm, about 1.5 mm to about 4.5 mm, about 1.5 mm to about 5 mm, about 1.5 mm to about 5.5 mm, about 1.5 mm to about 6 mm, about 1.5 mm to about 7 mm, about 1.5 mm to about 8 mm, about 1.5 mm to about 9 mm, about 1.5 mm to about 10 mm, about 2.5 mm to about 3.5 mm, about 2.5 mm to about 4.5 mm, about 2.5 mm to about 5 mm, about 2.5 mm to about 5.5 mm, about 2.5 mm to about 6 mm, about 2.5 mm to about 7 mm, about 2.5 mm to about 8 mm, about 2.5 mm to about 9 mm, about 2.5 mm to about 10 mm, about 3.5 mm to about 4.5 mm, about 3.5 mm to about 5 mm, about 3.5 mm to about 5.5 mm, about 3.5 mm to about 6 mm, about 3.5 mm to about 7 mm, about 3.5 mm to about 8 mm, about 3.5 mm to about 9 mm, about 3.5 mm to about 10 mm, about 4.5 mm to about 5 mm, about 4.5 mm to about 5.5 mm, about 4.5 mm to about 6 mm, about 4.5 mm to about 7 mm, about 4.5 mm to about 8 mm, about 4.5 mm to about 9 mm, about 4.5 mm to about 10 mm, about 5 mm to about 5.5 mm, about 5 mm to about 6 mm, about 5 mm to about 7 mm, about 5 mm to about 8 mm, about 5 mm to about 9 mm, about 5 mm to about 10 mm, about 5.5 mm to about 6 mm, about 5.5 mm to about 7 mm, about 5.5 mm to about 8 mm, about 5.5 mm to about 9 mm, about 5.5 mm to about 10 mm, about 6 mm to about 7 mm, about 6 mm to about 8 mm, about 6 mm to about 9 mm, about 6 mm to about 10 mm, about 7 mm to about 8 mm, about 7 mm to about 9 mm, about 7 mm to about 10 mm, about 8 mm to about 9 mm, about 8 mm to about 10 mm, or about 9 mm to about 10 mm. In some embodiments, the outer diameter of the scaffold is about 1.5 mm, about 2.5 mm, about 3.5 mm, about 4.5 mm, about 5 mm, about 5.5 mm, about 6 mm, about 7 mm, about 8 mm, about 9 mm, or about 10 mm. In some embodiments, the outer diameter of the scaffold is at least about 1.5 mm, about 2.5 mm, about 3.5 mm, about 4.5 mm, about 5 mm, about 5.5 mm, about 6 mm, about 7 mm, about 8 mm, or about 9 mm. In some embodiments, the outer diameter of the scaffold is at most about 2.5 mm, about 3.5 mm, about 4.5 mm, about 5 mm, about 5.5 mm, about 6 mm, about 7 mm, about 8 mm, about 9 mm, or about 10 mm. In some embodiments, the outer diameter of the scaffold is between 0.5 mm and 10 mm. In some embodiments, the outer diameter of the scaffold is between 0.1 mm and 10 mm. In some embodiments, the outer diameter of the scaffold is between 0.5 mm and 20 mm, between 0.5 mm and 30 mm, between 0.5 mm and 40 mm, or between 0.5 and 50 mm. In some embodiments, the outer diameter of the scaffold is between 0.5 mm and 20 mm.

The microchannels are formed of a biocompatible and biodegradable material, such as a biocompatible polymer. For example, a scaffold structure can comprise microchannels formed from biocompatible and biodegradable polymers, such as polyester polymers. Suitable biodegradable and biocompatible polymers for forming the microchannels include a polyethylene glycol, a gelatin, or a collagen, and derivatives, and mixtures thereof. In certain aspects, the biocompatible and biodegradable material is selected from a group of polymers consisting of poly(ethylene glycol) diacrylate, methacrylated gelatin, and methacrylated collagen, and combinations thereof. In other embodiments, the scaffold structure may be comprised of other polymeric materials such as polycaprolactone or acrylated polycaprolactone. In some embodiments, different portions of the scaffold structure may differ in material composition from other portions of the scaffold structure (for example, in one particular nonlimiting embodiment, the inner microchannels may be comprised of a mixture of methacrylated gelatin and polyethylene glycol while the outer sheath may be comprised of polycaprolactone). In some embodiments, scaffolds are prepared from mixtures of different polymerizable materials, such as those listed above. For example, a scaffold according to the present disclosure could be prepared from a mixture of poly(ethylene glycol) diacrylate and methacrylated gelatin. In some embodiments, the mixture will further comprise a suitable initiator for the polymerization reaction, such as, for example, lithium phenyl-2,4,6-trimethylbenzoylphosphinate. Any initiator suitable for the initiating the polymerization reaction may be employed. In some embodiments, the remainder of the mixture for preparing the scaffold is a suitable solvent, such as an aqueous solvent or buffer (e.g. phosphate buffered saline (PBS) or Dulbecco's phosphate-buffered saline (DPBS)).

In some embodiments, a scaffold is prepared from a mixture comprising poly(ethylene glycol) diacrylate and methacrylated gelatin. In some embodiments, a scaffold is prepared from about 25% poly(ethylene glycol) diacrylate (average size Mn 700) and about 1-7% methacrylated gelatin. In some embodiments, a scaffold is prepared from a mixture comprising about 15%, about 17.5%, about 20%, about 22.5%, about 25%, about 27.5%, about 30%, about 32.5%, or about 35% poly(ethylene glycol) diacrylate. In some embodiments, a scaffold is prepared from a mixture comprising about 22.5% to about 27.5%, about 20% to about 30%, about 17.5% to about 32.5%, or about 15% to about 35% poly(ethylene glycol) diacrylate. In some embodiments, a scaffold is prepared form a mixture comprising about 25% poly(ethylene glycol) diacrylate. In some embodiments, a scaffold is prepared form a mixture comprising about 15% to about 30% poly(ethylene glycol) diacrylate. In some embodiments, a scaffold is prepared form a mixture comprising about 20% to about 25% poly(ethylene glycol) diacrylate. In some embodiments, a scaffold is prepared form a mixture comprising about 15% to about 25% poly(ethylene glycol) diacrylate. In some embodiments, a scaffold is prepared form a mixture comprising about 20% to about 30% poly (ethylene glycol) diacrylate. In some embodiments, the average molecular weight of the poly(ethylene glycol) diacrylate is about Mn 550, about Mn 700, about Mn 1000, about Mn 2000, or about Mn 4000. In some embodiments, the average molecular weight of the poly(ethylene glycol) diacrylate is from about Mn 500 to about Mn 1000. In some embodiments, the average molecular weight of the poly(ethylene glycol) diacrylate is about Mn 700. In some embodiments, the average molecular weight of the poly(ethylene glycol) diacrylate is from about 100 Mn and 10000 Mn. In some embodiments, a scaffold is prepared from a mixture comprising about 1-7% methacrylated gelatin. In some embodiments, a scaffold is prepared from a mixture comprising about 1-7%, about 2-6%, or about 3-5% methacrylated gelatin. In some embodiments, a scaffold is prepared from a mixture comprising about 1-15% methacrylated gelatin. In some embodiments, a scaffold is prepared from a mixture comprising poly(ethylene glycol) diacrylate and methacrylated gelatin in a ratio of about 3:1, about 7:2, about 4:1, about 5:1, about 10:1, about 15:1, about 20:1, or about 25:1 (w/w). In some embodiments, a scaffold is prepared from a mixture comprising poly(ethylene glycol) diacrylate and methacrylated gelatin in a ratio from about 25:1 to about 3:1, from about 20:1 to about 7:2, from about 15:1 to about 4:1, or from about 10:1 to about 4:1 (w/w). In some embodiments, a scaffold comprises poly(ethylene glycol) diacrylate and methacrylated gelatin in a ratio of about 3:1, about 7:2, about 4:1, about 5:1, about 10:1, about 15:1, about 20:1, or about 25:1 (w/w). In some embodiments, a scaffold comprises poly(ethylene glycol) diacrylate and methacrylated gelatin in a ratio from about 25:1 to about 3:1, from about 20:1 to about 7:2, from about 15:1 to about 4:1, or from about 10:1 to about 4:1 (w/w).

In some embodiments, a scaffold is prepared from a mixture comprising poly (ethylene glycol) diacrylate and methacrylated collagen. In some embodiments a scaffold is prepared from about 25% poly(ethylene glycol) diacrylate (average size Mn 700) and about 2-10 mg/ml methacrylated collagen. In some embodiments, a scaffold is prepared from a mixture comprising about 15%, about 17.5%, about 20%, about 22.5%, about 25%, about 27.5%, about 30%, about 32.5%, or about 35% poly(ethylene glycol) diacrylate. In some embodiments, a scaffold is prepared from a mixture comprising about 22.5% to about 27.5%, about 20% to about 30%, about 17.5% to about 32.5%, or about 15% to about 35% poly(ethylene glycol) diacrylate In some embodiments, a scaffold is prepared form a mixture comprising about 25% poly(ethylene glycol) diacrylate. In some embodiments, a scaffold is prepared form a mixture comprising about 20% to about 30% poly(ethylene glycol) diacrylate. In some embodiments, a scaffold is prepared form a mixture comprising about 15% to about 30% poly(ethylene glycol) diacrylate. In some embodiments, a scaffold is prepared form a mixture comprising about 20% to about 25% poly(ethylene glycol) diacrylate. In some embodiments, a scaffold is prepared form a mixture comprising about 15% to about 25% poly(ethylene glycol) diacrylate. In some embodiments, a scaffold is prepared form a mixture comprising about 20% to about 30% poly (ethylene glycol) diacrylate. In some embodiments, the average molecular weight of the poly(ethylene glycol) diacrylate is about Mn 550, about Mn 700, about Mn 1000, about Mn 2000, or about Mn 4000. In some embodiments, the average molecular weight of the poly(ethylene glycol) diacrylate is from about Mn 500 to about Mn 1000. In some embodiments, the average molecular weight of the poly(ethylene glycol) diacrylate is about Mn 700. In some embodiments, the average size of the poly(ethylene glycol) diacrylate is from about 100 Mn and 10000 Mn. In some embodiments, a scaffold is prepared from a mixture comprising about 2-10 mg/ml, about 3-9 mg/mL, or about 4-8 mg/ml methacrylated collagen. In some embodiments, a scaffold is prepared from a mixture comprising about 1-15 mg/ml methacrylated collagen. In some embodiments, a scaffold is prepared from a mixture comprising poly(ethylene glycol) diacrylate and methacrylated collagen in a ratio from about 125:1 to about 25:1, about 100:1 to about 40:1, about 75:1 to about 50:1 (w/w). In some embodiments, a scaffold is prepared from a mixture comprising poly(ethylene glycol) diacrylate and methacrylated collagen in a ratio of about 125:1, about 100:1, about 75:1, about 50:1, about 40:1, or about 25:1 (w/w). In some embodiments, a scaffold comprises poly(ethylene glycol) diacrylate and methacrylated collagen in a ratio from about 125:1 to about 25:1, about 100:1 to about 40:1, about 75:1 to about 50:1 (w/w). In some embodiments, a scaffold comprises poly(ethylene glycol) diacrylate and methacrylated collagen in a ratio of about 125:1, about 100:1, about 75:1, about 50:1, about 40:1, or about 25:1 (w/w).

In certain aspects, the microchannels may be treated with a biofunctional agent or active ingredient; have different surface properties or surface roughness; or have surfaces with different moieties exposed, which can be useful in designing spatially guided cellular growth and in certain aspects to facilitate adhesion of cells or tissue or to promote release of biofunctional agents, which include biofunctional materials and active ingredients (e.g., pharmaceutical active ingredients), and the like, into the surrounding environment.

The biodegradable material forming the microchannel may dissolve, referring to physical disintegration, erosion, disruption and/or dissolution of a material and may include the resorption of such material by a living organism. In certain variations, biodegradable polymeric material may dissolve or erode upon exposure to a solvent comprising a high concentration of water, such as blood, serum, growth or culture media, bodily fluids, saliva, and the like. Thus, upon implantation, the material may dissolve or disintegrate into small pieces. For structural scaffold members, the dissolution rate (e.g., a rate at which the structural member is resorbed by surrounding cells) can be designed so that sufficient cellular growth occurs prior to the structure dissolving or disintegrating via the resorption process. In various embodiments, the tissue scaffold device is designed to have a degradation time or dissolution rate that coincides with an amount of time that permits adequate neural tissue regrowth through the scaffold to a target tissue in the subject. Depending upon the subject and the time needed for recuperation and regeneration of the tissue, by way of non-limiting example, the degradation time may be greater than or equal to about 1 month to less than or equal to about 3 years, greater than or equal to about 1 month to less than or equal to 1 year, and in certain variations, greater than or equal to about 1 month to less than or equal to 6 months. In this manner, the cellular scaffold structure supports and promotes cell growth, cell proliferation, cell differentiation, cell repair, and/or cell regeneration in three-dimensions, especially for neural tissue growth.

In certain aspects, the walls of the microchannels are porous. The pore size may be selected to promote substantially linear neural or axonal tissue growth along the longitudinal axis while avoiding cell growth through and across the microchannel walls. In some embodiments, the microchannels are made of a hydrogel, for example a mixture of poly(ethylene glycol) diacrylate and methacrylated gelatin or a mixture of poly(ethylene glycol) diacrylate and methacrylated collagen. Due to the properties of the microchannels comprising hydrogels provided herein, nutrients can be exchanged between the exterior of the scaffolds and the interior of the microchannel without the use of pores. The natural porosity of these materials allows flow of nutrients and oxygen through the microchannels walls to support cells growing through the lumen while preventing cell growth in undesired directions (for example, through the microchannels walls).

The walls of the microchannels optionally comprise a plurality of pores having an average pore size diameter of less than or equal to about 50 µm, optionally less than or equal to about 40 µm, optionally less than or equal to about 30 µm, optionally less than or equal to about 20 µm, and in certain variations, optionally less than or equal to about 10 µm. In certain aspects, the plurality of pores in the microchannel wall has an average pore size that eliminates line-of-site pores that could allow axons to grow between respective microchannels. Such pore sizes promote flow of oxygen and nutrients through the walls of the microchannel from the external surface to the internal surface to support cells growing within the open central lumen, while minimizing or preventing cells from being able to grow through the microchannel walls.

In other aspects, the present disclosure provides methods of making a biomimetic scaffold for promoting neural tissue growth by 3D printing. Scaffolds provided herein can be made using a variety of 3D printing techniques. Examples of 3D printing techniques which can be used to prepare 3D printed scaffolds include extrusion printing, inkjet printing, laser based-stereolithography, digital light processing stereolithography, and volumetric 3D printing (a.k.a. holographic 3D printing). In some embodiments, a biomimetic scaffold provided herein is prepared by digital light processing 3D printing. Additional method of 3D printing of biomimetic scaffolds are described in PCT/US2017/065857, which is hereby incorporated by reference in its entirety.

In other aspects, the biomimetic scaffolds provided herein can be made from other techniques, including casting, molding, electrospinning, embossing, or any other suitable method. Exemplary alternative methods for preparing biomimetic scaffolds are described in, for example, PCT/US2016/056104 and PCT/US2020/012966, each of which is incorporated by reference in its entirety.

The disclosed microchannel scaffolds disclosed herein promote cell growth, proliferation, differentiation, repair, and/or regeneration of tissue. In certain embodiments, the tissue is a neural tissue, such as axons.

In certain embodiments, suitable wall thicknesses of a microchannel wall are the smallest thicknesses possible that retain structural integrity to the channel. In certain aspects, the wall has a thickness of less than or equal to about 500 µm. In other aspects, the wall has a thickness of less than or equal to about 100 µm. Where wall thicknesses are greater than 100 µm, they can reduce the amount of space available within the open central lumen for axonal regeneration. In certain variations, the wall thickness may be greater than or equal to about 10 µm to less than or equal to about 100 µm, optionally greater than or equal to about 10 µm to less than or equal to about 70 µm, optionally greater than or equal to about 20 µm to less than or equal to about 70 µm, optionally greater than or equal to about 25 µm to less than or equal to about 67 µm, and in certain aspects, optionally greater than or equal to about 20 µm to less than or equal to about 50 µm. In certain other variations, the wall has a thickness of greater than or equal to about 10 µm to less than or equal to about 20 µm.

One particular advantage of the tissue scaffold design according to various aspects of the present disclosure is providing an overall open volume (e.g., open lumen volume, including the volume of open interstitial channels within sheath and open central lumen of microchannels) of greater than or equal to about 50 volume %, optionally greater than or equal to about 60 volume %, optionally greater than or equal to about 70 volume %, optionally greater than or equal to about 80 volume %, and in certain preferred aspects, optionally greater than or equal to about 90 open volume % of the overall scaffold volume. It should be noted that conventional scaffold designs were not able to achieve such high levels of open lumen volumes, which is believed to be particularly advantageous in supporting and promoting growth of healthy neural tissues having desirably high directional linearity and high signal fidelity.

In certain aspects, a diameter of each microchannel of the plurality of microchannels disposed within the sheath is selected to be the same (or substantially the same accounting for small dimensional variances during manufacturing), although in alternative variations, the diameters may intentionally vary between distinct microchannels of the plurality present in the sheath. As noted above, in variations where the plurality of microchannels have substantially the same diameter, an average inner diameter is optionally less than or equal to about 450 µm or any of the other ranges specified previously. Each microchannel may have an oval or spherical cross-sectional shape to form microcylinder shapes that create significant open interstitial volumes in interstitial channels, although in alternative variations, other shapes may be used. Where the plurality of microchannels has substantially the same diameters, they may be configured to be closely packed in an array within the sheath. Thus, each microchannel contacts another adjacent microchannel. The plurality of microchannels may be arranged within the sheath in a close-packed array that may create a honeycomb type of arrangement. In this manner, the tissue scaffolds of the present disclosure comprise discrete, linear, thin-walled, close-packed arrays of microchannels disposed within external protective sheath. A microchannel density may be varied in different embodiments, for example, the microchannel density may be greater than or equal to about 1 to less than or equal to about 300 microchannels/mm² in the scaffold. In certain variations, the microchannel density may be greater than or equal to about 10 to less than or equal to about 30 microchannels/mm². In another variation, the tissue scaffold may have a microchannel density of about 120 microchannels/mm². In some variations, the microchannel density is about 10 to about 300 microchannels/mm². In some variations, the microchannel density is about 10 to about 20, about 10 to about 30, about 10 to about 50, about 10 to about 100, about 10 to about 120, about 10 to about 150, about 10 to about 200, about 10 to about 300, about 20 to about 30, about 20 to about 50, about 20 to about 100, about 20 to about 120, about 20 to about 150, about 20 to about 200, about 20 to about 300, about 30 to about 50, about 30 to about 100, about 30 to about 120, about 30 to about 150, about 30 to about 200, about 30 to about 300, about 50 to about 100, about 50 to about 120, about 50 to about 150, about 50 to about 200, about 50 to about 300, about 100 to about 120, about 100 to about 150, about 100 to about 200, about 100 to about 300, about 120 to about 150, about 120 to about 200, about 120 to about 300, about 150 to about 200, about 150 to about 300, or about 200 to about 300 microchannels/mm². In some variations, the microchannel density is about 10, about 20, about 30, about 50, about 100, about 120, about 150, about 200, or about 300 microchannels/mm². In some variations, the microchannel density is at least about 10, about 20, about 30, about 50, about 100, about 120, about 150, or about 200 microchannels/mm². In some variations, the microchannel density is at most about 20, about 30, about 50, about 100, about 120, about 150, about 200, or about 300 microchannels/mm². In some embodiments, the number of microchannels in a single sheath can be from 7 to over 200 channels. In some variations, the number of microchannels in a single sheath is from about 7 to about 200 channels. In some variations, the number of microchannels in a single sheath is from about 7 to about 15, about 7 to about 25, about 7 to about 50, about 7 to about 75, about 7 to about 100, about 7 to about 150, about 7 to about 200, about 15 to about 25, about 15 to about 50, about 15 to about 75, about 15 to about 100, about 15 to about 150, about 15 to about 200, about 25 to about 50, about 25 to about 75, about 25 to about 100, about 25 to about 150, about 25 to about 200, about 50 to about 75, about 50 to about 100, about 50 to about 150, about 50 to about 200, about 75 to about 100, about 75 to about 150, about 75 to about 200, about 100 to about 150, about 100 to about 200, or about 150 to about 200 channels. In some variations, the number of microchannels in a single sheath is from about 7, about 15, about 25, about 50, about 75, about 100, about 150, or about 200 channels. In some variations, the number of microchannels in a single sheath is from at least about 7, about 15, about 25, about 50, about 75, about 100, or about 150 channels. In some variations, the number of microchannels in a single sheath is from at most about 15, about 25, about 50, about 75, about 100, about 150, or about 200 channels. In some embodiments, the number of microchannels in a single sheath is at most about 300, 400, 500, 750, or 1000 channels.

The sheath may be formed of a biocompatible and/or biodegradable material that may be the same as, or different from, the microchannels. Desirably, the sheath may have a similar porosity to the microchannels to promote flow and transport of nutrients to the microchannels, while minimizing or preventing cellular growth from an interior region through the wall of the sheath to an exterior region. The sheath is shown as a cylindrical tube shape with an oval or cylindrical cross-sectional shape; however, the sheath may have a variety of other shapes, so long as the microcylinders can be arranged in an array within the sheath. Thus, in certain aspects, the sheath may have other shapes, including a butterfly shape similar to that found in a human spinal column by way of non-limiting example. The sheath may have a length that is the same as the microcylinders or may be longer, such as an overhang, for additional protection and securing to a portion of a nerve or surrounding tissue (e.g., by anastomosing). In this manner, the tissue scaffold, including the sheath and microchannels can extend over any distance to match injuries of individual subjects/patients. Further, the actual shape or contour of the overall scaffold may be created to match the exact shape or contour of a given injury (this shape may be determined by conventional medical imaging methods such as MRI, CT, ultrasound, etc).

The scaffold can be filled with cells. These cells can be modified to express a growth factor or can be therapeutic in nature such as stem cells or Schwann cells.

A portion of nerve, such as a nerve end, of the subject may be damaged or severed, for example, a fully or partially lesioned nerve end caused by injury, disease, or surgery. In certain aspects, a portion of the nerve end may be surgically divided, sectioned, cut, and/or transected into one or more individual branches or fascicles that may be secured to a proximal end or distal end of the tissue scaffold. The one or more individual branches or fascicles of the nerve end may contact or be placed within one or more microchannels. The nerve end (or its individual branches or fascicles) can be secured via sutures, adhesives, or other known securing techniques to the proximal or distal ends of the sheath. Over a period of, for example, several months, the neural tissue originating from the nerve end can grow along the longitudinal axis of each microchannel and reinnervate any neural targets at the opposite end of the tissue scaffold. The tissue scaffolds according to various aspects of the present teachings thus facilitate neural tissue growth through the open central lumens of the plurality of microchannels from a first end to a second opposite end of the scaffold.

As will be appreciated by those of skill in the art, while the design of the inventive tissue scaffolds is particularly suitable for promoting neural tissue growth, in alternative variations, the tissue scaffold may be used for other types of tissue growth.

In other aspects, surfaces of the walls of microchannels may be coated with a biofunctional agent to promote cell growth, regeneration, differentiation, proliferation, and/or repair, for example. By "promoting" cell growth, cell proliferation, cell differentiation, cell repair, or cell regeneration, it is meant that a detectable increase occurs in either a rate or a measurable outcome of such processes occurs in the presence of the biofunctional agent as compared to a cell or organism's process in the absence of such a biofunctional agent, for example, conducting such processes naturally. By way of example, as appreciated by those of skill in the art, promoting cell growth in the presence of a biofunctional agent may increase a growth rate of target cells or increase a total cell count of the target cells, when compared to cell growth or cell count of the target cells in the absence of such a biofunctional agent.

As used herein, "biofunctional agent" refers to a molecule which promotes cell growth, cell adhesion, cell proliferation, cell differentiation, cell repair, and/or cell regeneration by increasing a measurable process result (e.g., measuring total cell counts for cell generation or cell regeneration, measuring the rates or qualitative outcome of cell proliferation, cell differentiation, or cell repair rates). In some embodiments, a biofunctional agent disclosed herein promotes a regenerative process by greater than or equal to about 25% as compared to the result of the process in the absence of the biofunctional agent, optionally increasing by greater than or equal to about 30%, optionally increasing by greater than or equal to about 35%, optionally increasing by greater than or equal to about 40%, optionally increasing by greater than or equal to about 45%, optionally increasing by greater than or equal to about 50%, optionally increasing by greater than or equal to about 55%, optionally increasing by greater than or equal to about 60%, optionally increasing by greater than or equal to about 65%, optionally increasing by greater than or equal to about 70%, optionally increasing by greater than or equal to about 75%, optionally increasing by greater than or equal to about 80%, optionally increasing by greater than or equal to about 85%, optionally increasing by greater than or equal to about 90%, and in certain aspects, optionally increasing by greater than or equal to about 95%.

Exemplary biofunctional agents include, but are not limited to fibronectin, keratin, laminin, collagen, a growth factor, and/or a stem cell-promoting factor. Exemplary growth factors include brain derived neurotrophic factor (BDNF), nerve growth factor, glial cell-derived neurotrophic factor (GDNF), and neurotrophin-3 (NT-3). In some embodiments, the growth factor is BDNF. In some embodiments, the growth factor is nerve growth factor. In some embodiments, the growth factor is GDNF. In some embodiments, the growth factor is NT-3.

Such a biofunctional agent may be introduced after the microchannels are formed, for example, by coating, infusing, or otherwise incorporating the biofunctional agent onto one of more surfaces (e.g., internal surface) of the microchannel wall. In certain aspects, a surface of the porous wall has a coating comprising a material for promoting growth of the neural tissue selected from the group consisting of: fibronectin, keratin, laminin, collagen, and combinations and equivalents thereof. In certain embodiments, the walls may be coated with fibronectin, which has been found after screening over a dozen compounds to be particularly advantageous with the biocompatible polymers forming the microchannel walls to optimize cell and axon attachment.

The present technology thus enables a major advance over existing technologies in surgical repair of injured peripheral nerves. These existing devices consist of only a single open channel (not divided into individual microchannels) in which axons frequently diverge from linear paths, reducing the number of axons that reach the distal end of the scaffold and contribute to nerve repair. Simpler designs like those commercially available more commonly result in painful neuromas and lack of functional improvement because of axon misguidance. Additionally, the properties of materials out of which existing scaffolds have been fabricated do not adequately support cell and axon attachment. Based on empirical observation after implanting and testing hydrogel nerve regeneration scaffolds, hydrogel-based materials do not exhibit adequate strength to enable the fabrication of thin (<50 µm) wall scaffolds. Yet based on calculations, it appears that wall thicknesses of less than 50 microns are necessary to achieve >80% lumen volume scaffolds that adequately support and promote neural tissue growth. Thus, currently available hydrogel based materials cannot provide scaffolds having adequate strength with advantageous open lumen volume provided by certain aspects of the present teachings. Conversely, the materials provided herein, for example hydrogels comprising a mixture of poly(ethylene glycol) methacrylate and methacrylated gelatin or hydrogels comprising a mixture of poly(ethylene glycol) methacrylate and methacrylated collagen, are mechanically engineered to be stable *in vivo.* In some embodiments, the use of 3D printing (e.g. digital light processing or other suitable method) allows for high resolution in the printing of the microchannel walls. This high resolution, in some embodiments in conjunction with the materials provided herein, allows for a scaffold to possess the required strength to remain stable *in vivo,* In some embodiments, the high resolution 3D printing allows for construction of scaffolds with wall thicknesses as low as 10 micron.

The present tissue scaffold devices are superior in providing a multi-lumen design that enhances nerve guidance, thereby increasing the total number of axons that regenerate successfully. As a result, such tissue scaffold devices work over long nerve gaps and in more proximal nerve injuries, thereby addressing a great unmet medical need. Further, the tissue scaffolds according to the present disclosure are made from biocompatible and biodegradable materials, such as poly(ethylene glycol) diacrylate, methacryated gelatin, methacrylated collagen, polycaprolactone, or acrylated polycaprolactone, with optimized porosity and surface roughness, providing superior cell adhesion levels and directional cell growth while exhibiting significantly reduced inflammatory response in vivo after implantation. When tested *in vivo,* the devices of the present disclosure are biocompatible.

In this manner, the tissue scaffold devices according to certain aspects of the present disclosure enable one or more of the following unique features or advantages: a close-packed array of linear microchannels that emulate native nerve organization, microchannels having significant and customizable lengths; hexagonal microchannels to maximize the number of channels within a sheath; thin-walled microchannels to maximize open volume; high open lumen volumes; the scaffold devices comprise biocompatible materials; an ability to control mechanical properties to optimize for strength to minimize wall thickness and sutureability as an outer sheath tube; an ability to control scaffold and sheath porosity to prevent axon penetration while allowing permeation of oxygen and other nutrients; an ability to modify microchannel surface properties to enable cell attachment; a single one-piece sheath and scaffold construction facilitating ease of implantation enabling secure apposition between nerve stumps and scaffold walls; and finally low material and fabrication cost.

Further areas of applicability will become apparent from the description provided herein. The description and specific examples in this summary are intended for purposes of illustration only and are not intended to limit the scope of the present disclosure.

### EXAMPLES

### Example 1. Multichannel Scaffold for Nerve Injury Repair in Rat Model

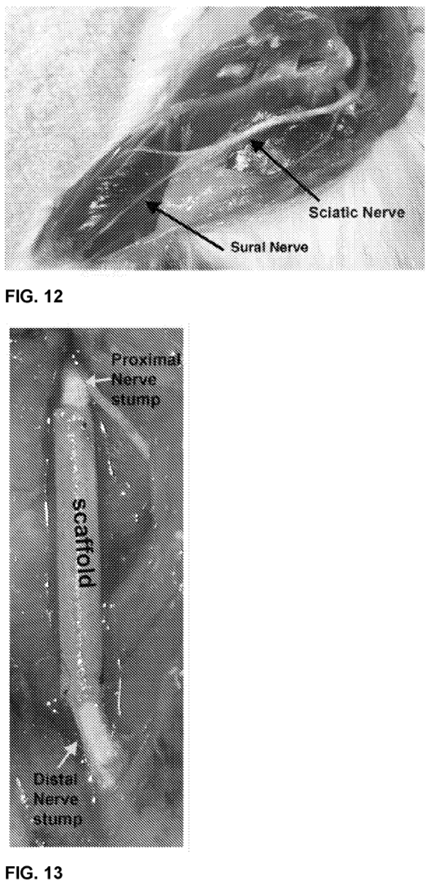

In some embodiments, devices are produced from porous PCL and comprise linear microchannels. The entire device has an inner diameter of 1.6 mm, has a length of 10 mm, and has a 1-mm overhang of the outer sheath on either side (e.g., for suturing in place in a subject). To assess the efficacy of these devices for nerve repair, devices are tested in the rat sciatic nerve model. An image of an intact sciatic nerve in a rat is shown in FIG. 12. Animals are housed (e.g., 2-3 per cage) with free access to food and water in facilities approved by the American Association for the Accreditation of Laboratory Animal Care. All animal studies are carried out according to NIH guidelines for laboratory animal care and safety, adhering to protocols approved by the Institutional Animal Care and Use Committee of the VA Healthcare System, San Diego.

To implant devices (n = 6), animals are deeply anesthetized (e.g., using ketamine (25 mg/mL), xylazine (1300 mg/mL), and acepromazine (0.25 mg/mL)) before making a 20-mm long incision on the right lateral thigh. The right sciatic nerve trunk is exposed via a lateral gluteal muscle dissection. Epineural connective tissue around the nerve trunk is separated with microscissors and a 6.0-mm long nerve segment is excised. After tissue retraction, the severed nerve stumps are further separated to about 15 mm; they are protected and hydrated with physiological saline. Devices are positioned and attached to the nerve, at either end, using 9-0 Ethicon suture. Devices are positioned to avoid tension at the interfaces of the device and nerve site. Following implantation, muscles are sutured using 5-0 suture and the skin is closed using clips. Antibiotics and analgesics (e.g., banamine (1 mg/kg) and ampicillin (0.2 mg/kg) in Ringer's lactate) are administered for the first 3 days to facilitate recovery from surgery. After 4 weeks, devices are harvested. Animals are perfused with 4% paraformaldehyde (PFA) and the tissue is removed and post-fixed in PFA for another 24 hours followed by 48 hours in 30% sucrose.

After 4 weeks, observations are expected to indicate no sign of degradation of the device. To assess the regeneration of nerves across the lesion site, immunolabeling is performed on histological sections. The tissue is processed for: 1) axon labeling, e.g., to assess axon regeneration through and beyond the injury site (NF200); and 2) Schwann cells (S100).

The microchannels of the device produce an aligned growth of neurites through the length of the scaffold, with nerves exiting the distal side of the implant. Unlike more traditional manufacturing methods (e.g., dip-coating), the high open lumen volume of the devices provided herein allows a greater number of neurons to regenerate due to the reduction of volume taken up by the pore walls. Accordingly, the technology provides for the faster healing of nerve lesions with better functional recovery.

### Example 2. Multichannel Scaffold for Nerve Injury Repair in Rat Model

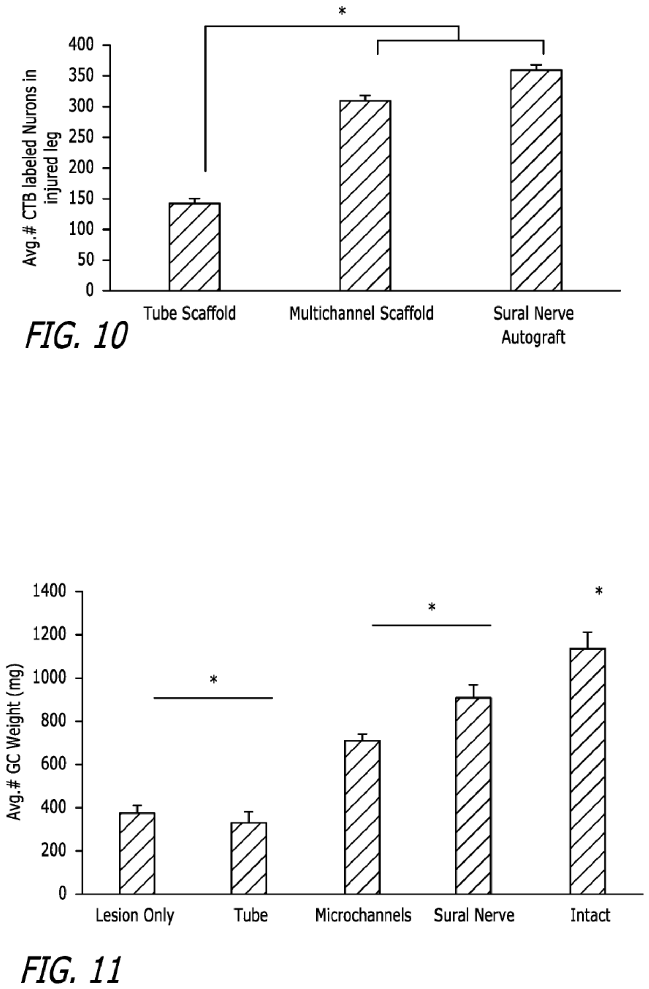

Multichannel scaffolds prepared by an embossing method were implanted in a 1 cm-long defect in rat sciatic nerve and compared to sural nerve autograft or open tube implant. The scaffolds used in this example had 8 microchannels which were each approximately 200 micron in diameter. The scaffolds were 1cm in length with an outer diameter of 1.7mm. An example image of the scaffold implanted in a rat is shown in FIG. 13. Four weeks post-implant multichannel scaffolds support linear alignment and accelerated regeneration of axons across the injury site. Six months post implant multichannel scaffolds showed improved connectivity between spinal cord and gastrocnemius muscle, compared to open tube treatment and comparable to the autograft. In addition, multichannel scaffolds support increased muscle mass, double the amount of increased muscle mass compared to lesion-only or open tube treatments, and comparable to autograft. The multichannel scaffold shown in FIG. 9B shows superior axonal alignment and faster rate of regeneration across a 1 cm sciatic nerve gap in the rat (shown 4 weeks post injury) compared to an open tube scaffold at the same time point (FIG. 9A). FIG. 10 shows improved connectivity between spinal cord motor neurons and muscle, assessed by injection of retrograde tracer (Cholera Toxin B) into gastrocnemius muscle six months after nerve repair. FIG. 11 shows significantly improved muscle mass. Statistically, the multichannel scaffold is as effective as a sural nerve autograft. N=11 animals per group.

The biomimetic scaffold with hexagonal microchannels disclosed herein has improved anatomical and electrophysiological connectivity across a sciatic nerve injury site and supports recovery of motor function.

Unless otherwise indicated, all numbers expressing quantities of ingredients, properties such as molecular weight, reaction conditions, and so forth used in the specification and claims are to be understood as being modified in all instances by the term "about." As used herein the terms "about" and "approximately" means within 10 to 15%, preferably within 5 to 10%. Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

Furthermore, numerous references have been made to patents and printed publications throughout this specification. Each of the above-cited references and printed publications are individually incorporated herein by reference in their entirety.

When a component, element, or layer is referred to as being "on," "engaged to," "connected to," or "coupled to" another element or layer, it may be directly on, engaged, connected or coupled to the other component, element, or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various steps, elements, components, regions, layers and/or sections, these steps, elements, components, regions, layers and/or sections should not be limited by these terms, unless otherwise indicated. These terms may be only used to distinguish one step, element, component, region, layer or section from another step, element, component, region, layer or section. Terms such as "first," "second," and other numerical terms when used herein do not imply a sequence or order unless clearly indicated by the context. Thus, a first step, element, component, region, layer or section discussed below could be termed a second step, element, component, region, layer or section without departing from the teachings of the example embodiments.

Spatially or temporally relative terms, such as "before," "after," "inner," "outer," "beneath," "below," "lower," "above," "upper," and the like, may be used herein for ease of description to describe one element or feature's relationship to another element(s) or feature(s) as illustrated in the figures. Spatially or temporally relative terms may be intended to encompass different orientations of the device or system in use or operation in addition to the orientation depicted in the figures.

Throughout this disclosure, the numerical values represent approximate measures or limits to ranges to encompass minor deviations from the given values and embodiments having about the value mentioned as well as those having exactly the value mentioned. Other than in the working examples provided at the end of the detailed description, all numerical values of parameters (e.g., of quantities or conditions) in this specification, including the appended claims, are to be understood as being modified in all instances by the term "about" whether or not "about" actually appears before the numerical value. "About" indicates that the stated numerical value allows some slight imprecision (with some approach to exactness in the value; approximately or reasonably close to the value; nearly). If the imprecision provided by "about" is not otherwise understood in the art with this ordinary meaning, then "about" as used herein indicates at least variations that may arise from ordinary methods of measuring and using such parameters. For example, "about" may comprise a variation of less than or equal to 5%, optionally less than or equal to 4%, optionally less than or equal to 3%, optionally less than or equal to 2%, optionally less than or equal to 1%, optionally less than or equal to 0.5%, and in certain aspects, optionally less than or equal to 0.1%.

In addition, disclosure of ranges includes disclosure of all values and further divided ranges within the entire range, including endpoints and sub-ranges given for the ranges.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

### NUMBERED EMBODIMENTS

The following embodiments recite nonlimiting permutations of combinations of features disclosed herein. Other permutations of combinations of features are also contemplated. In particular, each of these numbered embodiments is contemplated as depending from or relating to every previous or subsequent numbered embodiment, independent of their order as listed.

Embodiment 1. A nerve repair scaffold comprising: a sheath having a proximal end and a distal end, the sheath housing a plurality of microchannels traversing the sheath from the proximal end to the distal end, wherein the microchannels are configured to allow growth of nerve tissue; and a first overhang at the proximal end and a second overhang at the distal end, wherein the first overhang and the second overhang are configured for suturing of nerve tissue.

Embodiment 2. The nerve repair scaffold of embodiment 1, wherein the microchannels are hexagonal, round, triangular, rectangular, square, pentagonal, heptagonal, octagonal, nonagonal, decagonal, elliptical, or trapezoidal in shape.

Embodiment 3. The nerve repair scaffold according to embodiment 2, wherein the microchannels are hexagonal in shape.

Embodiment 4. The nerve repair scaffold according to embodiment 2, wherein the microchannels are round in shape.

Embodiment 5. The nerve repair scaffold according to any one of embodiments 1-4, wherein the scaffold comprises about 7 to about 200 microchannels.

Embodiment 6. The nerve repair scaffold according to embodiment 5, wherein the scaffold comprises 5-15 microchannels.

Embodiment 7. The nerve repair scaffold according to any one of embodiments 1-6, wherein the microchannel density is about 10 to about 300 microchannels/mm².

Embodiment 8. The nerve repair scaffold according to any one of embodiments 1-7, wherein the length of the scaffold is about 0.5 cm to about 15 cm.

Embodiment 9. The nerve repair scaffold according to any one of embodiments 1-8, wherein the outer diameter is about 1.5 mm to about 10 mm.

Embodiment 10. The nerve repair scaffold according to any one of embodiments 1-9, wherein each microchannel has an inner diameter from about 150 µm to about 250 µm.

Embodiment 11. The nerve repair scaffold according to any one of embodiments 1-10, wherein each microchannel has a wall thickness of about 10 µm to about 60 µm.

Embodiment 12 The nerve repair scaffold according to any one of embodiments 1-11 wherein each microchannel is the same size.

Embodiment 13. The nerve repair scaffold according to any one of embodiments 1-12, wherein the scaffold comprises microchannels of different sizes.

Embodiment 14. The nerve repair scaffold according to any one of embodiments 1-13, wherein the first and the second overhangs are independently from about 0.1 mm to about 3 mm in length.

Embodiment 15. The nerve repair scaffold according to any one of embodiments 1-14, wherein the first and the second overhangs have a thickness independently from about 0.1 mm to about 3 mm.

Embodiment 16. The nerve repair scaffold according to any one of embodiments 1-15, wherein the scaffold is formed from a biocompatible material selected from poly(ethylene glycol) diacrylate, methacrylated gelatin, methacrylated collagen, polycaprolactone, and acrylated polycaprolactone, or any combination thereof.

Embodiment 17. The nerve repair scaffold according to embodiment 16, wherein the average molecular weight of the poly(ethylene glycol) diacrylate is from about Mn 500 to about Mn 1000.

Embodiment 18. The nerve repair scaffold according to embodiment 17, wherein the average size of the poly(ethylene glycol) diacrylate is about Mn 700.

Embodiment 19. The nerve repair scaffold according to any one of embodiments 16-18, wherein the scaffold is formed from a mixture comprising poly(ethylene glycol) diacrylate and methacrylated gelatin.

Embodiment 20. The nerve repair scaffold according to embodiment 19, wherein the scaffold is prepared from about 25% poly(ethylene glycol) diacrylate and about 1-7% methacrylated gelatin.

Embodiment 21. The nerve repair scaffold according to embodiment 19, wherein the ratio of poly(ethylene glycol) diacrylate to methacrylated gelatin in the scaffold is from about 25:1 to about 3:1.

Embodiment 22. The nerve repair scaffold according to any one of embodiments 16-18, wherein the scaffold is formed from a mixture comprising poly(ethylene glycol) diacrylate and methacrylated collagen.

Embodiment 23. The nerve repair scaffold according to embodiment 22, wherein the scaffold is prepared from about 25% poly(ethylene glycol) diacrylate and about 2-10 mg/ml methacrylated gelatin.

Embodiment 24. The nerve repair scaffold according to embodiment 22, wherein the ratio of poly(ethylene glycol) diacrylate to methacrylated gelatin in the scaffold is from about 125:1 to about 25:1 (w/w).

Embodiment 25. The nerve repair scaffold according to any one of embodiments 1-24, wherein the scaffold further comprises a biofunctional agent.

Embodiment 26. The nerve repair scaffold according to embodiment 25, wherein the biofunctional agent is coated on a wall of a microchannel or incorporated into a wall of a microchannel.

Embodiment 27. The nerve repair scaffold according to embodiment 25 or 26, wherein the biofunctional agent comprises fibronectin, collagen, laminin, keratin, a growth factor, or a stem cell-promoting factor.

Embodiment 28. The nerve repair scaffold according to embodiment 27, wherein the growth factors is brain derived neurotrophic factor, nerve growth factor, glial cell-derived neurotrophic factor, or neurotrophin-3.

Embodiment 29. The nerve repair scaffold according to any one of embodiments 1-28, wherein the scaffold is further filled with cells.

Embodiment 30. The nerve repair scaffold according to embodiment 29, wherein the cells are stem cells or Schwann cells.

Embodiment 31. The nerve repair scaffold according to any one of embodiments 1-30, wherein the scaffold comprises an open volume of greater than or equal to about 70%.

Embodiment 32. The nerve repair scaffold according to any one of embodiments 1-31, wherein the scaffold is 3D printed.

Embodiment 33. A nerve repair scaffold comprising: a sheath having a proximal end and a distal end, the sheath housing a plurality of microchannels traversing the sheath from the proximal end to the distal end, wherein the microchannels are configured to allow growth of nerve tissue, and wherein at least one of the microchannel walls comprises a biofunctional agent incorporated into the microchannel wall.

Embodiment 34 The nerve repair scaffold according to embodiment 33, wherein the biofunctional agent promotes cell growth, regeneration, differentiation, proliferation, repair, or any combination thereof.

Embodiment 35. The nerve repair scaffold according to embodiment 33 or 34, wherein the biofunctional agent comprises fibronectin, keratin, laminin, collagen, a growth factor, or a stem cell-promoting factor.

Embodiment 36. The nerve repair scaffold according to any one of embodiments 33-35, wherein the growth factor is brain derived neurotrophic factor, nerve growth factor, glial cell- derived neurotrophic factor, or neurotrophin-3, or any combination thereof.

Embodiment 37. The nerve repair scaffold according to any one of embodiments 33-36, further comprising a first overhang at the proximal end and a second overhang at the distal end, wherein the first overhang and the second overhang are configured for suturing of nerve tissue.

Embodiment 38. The nerve repair scaffold according to any one of embodiments 33-37, wherein the scaffold is further filled with cells.

Embodiment 39. The nerve repair scaffold according to any one of embodiments 33-38, wherein each of the microchannels has an open dimeter of about 200 µm to about 500 µm.

Embodiment 40. The nerve repair scaffold according to any one of embodiments 33-39, wherein the microchannel density is from about 10 to about 30 microchannels/mm².

Embodiment 41. The nerve repair scaffold according to any one of embodiments 33-40, wherein the scaffold is prepared from about 20-30% poly(ethylene glycol) diacrylate and about 1-7% methacrylated gelatin.

Embodiment 42. The nerve repair scaffold according to any one of embodiments 33-41, wherein the ratio of poly(ethylene glycol) diacrylate to methacrylated gelatin in the scaffold is from about 25:1 to about 3:1.

Embodiment 43. The nerve repair scaffold according to any one of embodiments 33-40, wherein the scaffold is prepared from about 25% poly(ethylene glycol) diacrylate and about 2-10 mg/mL methacrylated collagen.

Embodiment 44. The nerve repair scaffold according to any one of embodiments 33-40 or 43, wherein the ratio of poly(ethylene glycol) diacrylate to methacrylated collagen in the scaffold is from about 125:1 to about 25:1.

Embodiment 45. The nerve repair scaffold according to any one of embodiments 33-44, wherein the plurality of microchannels comprises from about 7 to about 200 microchannels.

Embodiment 46. The nerve repair scaffold according to any one of embodiments 33-45, wherein the microchannel walls have a thickness of greater than or equal to about 10 µm to less than or equal to about 60 µm.

Embodiment 47. The nerve repair scaffold according to any one of embodiments 33-46, wherein the scaffold is from 0.5 mm to 15 cm in length.

Embodiment 48. The nerve repair scaffold according to any one of embodiments 33-47, wherein the scaffold is from 5 cm to 10 cm in length.

Embodiment 49. The nerve repair scaffold according to any one of embodiments 33-48, wherein the scaffold comprises an open volume of greater than or equal to about 70%.

Embodiment 50. The nerve repair scaffold according to any one of embodiments 33-49, wherein the scaffold is 3D printed.

Embodiment 51. A nerve repair scaffold comprising: a sheath having a proximal end and a distal end, the sheath housing a plurality of microchannels traversing the sheath from the proximal end to the distal end, wherein the microchannels are configured to allow growth of nerve tissue; a first overhang at the proximal end and a second overhang at the distal end, wherein the first overhang and the second overhang are configured for suturing of nerve tissue; wherein the scaffold further comprises a biofunctional agent; wherein each of the microchannels have an open dimeter of about 200 µm to about 350 µm; and wherein the scaffold is prepared from about 15% to about 25% poly(ethylene glycol) diacrylate and about 1-7% methacrylated gelatin.

Embodiment 52. The nerve repair scaffold according to embodiment 51, wherein the biofunctional agent is incorporated into at least one microchannel wall.

Embodiment 53. The nerve repair scaffold according to embodiment 51, wherein the biofunctional agent is coated on a microchannel wall.

Embodiment 54. The nerve repair scaffold according to any one of embodiments 51-53, wherein the biofunctional agent comprises fibronectin, keratin, laminin, collagen, a growth factor, or a stem cell-promoting factor.

Embodiment 55. The nerve repair scaffold according to any one of embodiments 51-54, wherein the growth factor is brain derived neurotrophic factor, nerve growth factor, glial cell- derived neurotrophic factor, or neurotrophin-3, or any combination thereof.

Embodiment 56. The nerve repair scaffold according to any one of embodiments 51-55, wherein the outer diameter of the scaffold is from about 1.5 mm to about 10 mm.

Embodiment 57. The nerve repair scaffold according to any one of embodiments 51-56, wherein the scaffold is from 0.5 mm to 10 cm in length.

Embodiment 58. The nerve repair scaffold according to any one of embodiments 51-57, wherein the scaffold is from 5 cm to 15 cm in length.

Embodiment 59. The nerve repair scaffold according to any one of embodiments 51-58, wherein the plurality of microchannels comprises from about 7 to about 200 microchannels.

Embodiment 60. The nerve repair scaffold according to any one of embodiments 51-59, wherein the microchannels are a hexagonal or round shape, or a combination thereof.

Embodiment 61. The nerve repair scaffold according to any one of embodiments 51-60, wherein the microchannel density is from about 10 to about 30 microchannels/mm².

Embodiment 62. The nerve repair scaffold according to any one of embodiments 51-61, wherein the scaffold comprises an open volume of greater than or equal to about 80%.

Embodiment 63. The nerve repair scaffold according to any one of embodiments 51-62, wherein the overhang has a length from about 0.1 mm to about 3 mm.

Embodiment 64. The nerve repair scaffold according to any one of embodiments 51-63, wherein the microchannel walls have a thickness of greater than or equal to about 10 µm to less than or equal to about 50 µm.

Embodiment 65. The nerve repair scaffold according to any one of embodiments 51-64, wherein the microchannels are filled with stem cells or Schwann cells.

Embodiment 66. The nerve repair scaffold according to any one of embodiments 51-65, wherein the nerve repair scaffold is 3D-printed.

Embodiment 67. A method of restoring nerve function comprising implanting the nerve repair scaffold of any one of embodiments 1-66 into a nerve injury site in a subject in need thereof, thereby allowing restoration of nerve function across the injury site.

Embodiment 68. The method of embodiment 67, wherein the nerve is a peripheral nerve.

Embodiment 69. The method of embodiment 67 or 68, wherein the nerve is fully or partially lesioned.

Embodiment 70. The method of any one of embodiments 67-69, wherein the nerve injury was caused by physical injury, disease, or surgery.

Embodiment 71. The method of any one of embodiments 67-70, wherein the nerve injury site comprises a gap between nerve ends from about 0.5 mm to about 10 cm.

Embodiment 72. The method of any one of embodiments 67-71, wherein the nerve injury site comprises a gap between nerve ends from about 5 cm to about 10 cm.

Embodiment 73. The method of any one of embodiments 67-72, wherein implanting the nerve repair scaffold comprises suturing nerve ends to the proximal end and distal end of the nerve repair scaffold.
The following numbered examples are also disclosed.
1. A nerve repair scaffold comprising:
   a sheath having a proximal end and a distal end, the sheath housing a plurality of microchannels traversing the sheath from the proximal end to the distal end, wherein the microchannels are configured to allow growth of nerve tissue; and
   a first overhang at the proximal end and a second overhang at the distal end, wherein the first overhang and the second overhang are configured for suturing of nerve tissue.
2. The nerve repair scaffold of example 1 , wherein the microchannels are hexagonal, round, triangular, rectangular, square, pentagonal, heptagonal, octagonal, nonagonal, decagonal, elliptical, or trapezoidal in shape.
3. The nerve repair scaffold according to example 1 or 2, wherein the scaffold comprises about 7 to about 200 microchannels.
4. The nerve repair scaffold according to any one of examples 1-3, wherein the length of the scaffold is about 0.5 cm to about 15 cm.
5. The nerve repair scaffold according to any one of examples 1-4, wherein the outer diameter is about 1.5 mm to about 10 mm.
6. The nerve repair scaffold according to any one of examples 1-5, wherein each microchannel has an inner diameter from about 150 µm to about 250 µm.
7. The nerve repair scaffold according to any one of examples 1-6, wherein each microchannel has a wall thickness of about 10 µm to about 60 µm.
8. The nerve repair scaffold according to any one of examples 1-7, wherein the scaffold is formed from a biodegradable material selected from polyethylene glycol) diacrylate, methacrylated gelatin, methacrylated collagen, polycaprolactone, and acrylated polycaprolactone, or any combination thereof.
9. The nerve repair scaffold according to any one of examples 1-8, wherein the scaffold is formed from a mixture comprising polyethylene glycol) diacrylate and methacrylated gelatin.
10. The nerve repair scaffold according to example 9, wherein the scaffold is prepared from about 25% polyethylene glycol) diacrylate and about 1-7% methacrylated gelatin.
11. The nerve repair scaffold according to example 8, wherein the scaffold is formed from a mixture comprising polyethylene glycol) diacrylate and methacrylated collagen.
12. The nerve repair scaffold according to any one of examples 1-1 1, wherein the scaffold further comprises a biofunctional agent.
13. The nerve repair scaffold according to example 12, wherein the biofunctional agent comprises fibronectin, collagen, laminin, keratin, a growth factor, or a stem cell-promoting factor.
14. The nerve repair scaffold according to any one of examples 1-13, wherein the scaffold comprises an open volume of greater than or equal to about 70%.
15. The nerve repair scaffold according to any one of examples 1-14, wherein the scaffold is 3D printed.
16. A nerve repair scaffold comprising:
   a sheath having a proximal end and a distal end, the sheath housing a plurality of microchannels traversing the sheath from the proximal end to the distal end, wherein the microchannels are configured to allow growth of nerve tissue, and
   wherein at least one of the microchannel walls comprises a biofunctional agent incorporated into the microchannel wall.
17. The nerve repair scaffold according to example 16, wherein the biofunctional agent comprises fibronectin, keratin, laminin, collagen, a growth factor, or a stem cell-promoting factor.
18. The nerve repair scaffold according to example 17, wherein the growth factor is brain derived neurotrophic factor, nerve growth factor, glial cell- derived neurotrophic factor, or neurotrophin-3, or any combination thereof.
19. The nerve repair scaffold according to any one of examples 16-18, wherein each of the microchannels has an open dimeter of about 200 µm to about 500 µm.
20. The nerve repair scaffold according to any one of examples 16-19, wherein the scaffold is prepared from about 20% to about 30% polyethylene glycol) diacrylate and about 1-7% methacrylated gelatin.
21. The nerve repair scaffold according to any one of examples 16-20, wherein the scaffold is prepared from about 25% polyethylene glycol) diacrylate and about 2-10 mg/ml_ methacrylated collagen.
22. The nerve repair scaffold according to any one of examples 16-21 , wherein the scaffold is from 0.5 mm to 15 cm in length.
23. The nerve repair scaffold according to any one of examples 16-22, wherein the scaffold comprises an open volume of greater than or equal to about 70%.
24. The nerve repair scaffold according to any one of examples 16-23 wherein the scaffold is 3D printed.
25. A nerve repair scaffold comprising:
   a sheath having a proximal end and a distal end, the sheath housing a plurality of microchannels traversing the sheath from the proximal end to the distal end, wherein the microchannels are configured to allow growth of nerve tissue;
   a first overhang at the proximal end and a second overhang at the distal end, wherein the first overhang and the second overhang are configured for suturing of nerve tissue; wherein the scaffold further comprises a biofunctional agent;
   wherein each of the microchannels have an open dimeter of about 200 µm to about 350 µm; and
   wherein the scaffold is prepared from about 15% to about 25% polyethylene glycol) diacrylate and about 1-7% methacrylated gelatin.
26. The nerve repair scaffold according to example 25, wherein the biofunctional agent is incorporated into at least one microchannel wall.
27. The nerve repair scaffold according to example 25 or 26, wherein the biofunctional agent comprises fibronectin, keratin, laminin, collagen, a growth factor, or a stem cell-promoting factor.
28. The nerve repair scaffold according to any one of examples 25-27, wherein the scaffold is from 0.5 mm to 15 cm in length.
29. The nerve repair scaffold according to any one of examples 25-28, wherein the microchannels are a hexagonal or round shape, or a combination thereof.
30. The nerve repair scaffold according to any one of examples 25-29, wherein the scaffold comprises an open volume of greater than or equal to about 70%.
31. The nerve repair scaffold according to any one of examples 25-30, wherein the nerve repair scaffold is 3D-printed
32 A method of restoring nerve function comprising implanting the nerve repair scaffold of any one of examples 1-31 into a nerve injury site in a subject in need thereof, thereby allowing restoration of nerve function across the injury site.
33. The method according to example 32, wherein the nerve is a peripheral nerve.

## Claims

1. A nerve repair scaffold comprising:
a sheath having a proximal end and a distal end, the sheath housing a plurality of microchannels traversing the sheath from the proximal end to the distal end, wherein the microchannels are configured to allow growth of nerve tissue; and
a first overhang at the proximal end and a second overhang at the distal end.

2. The nerve repair scaffold according to claim 1, wherein the scaffold comprises about 7 to about 200 microchannels.

3. The nerve repair scaffold according to any one of claims 1 or 2, wherein the length of the scaffold is about 0.5 cm to about 15 cm.

4. The nerve repair scaffold according to any one of claims 1-3, wherein the outer diameter is about 1.5 mm to about 10 mm.

5. The nerve repair scaffold according to any one of claims 1-4, wherein each microchannel has an inner diameter from about 150 µm to about 250 µm.

6. The nerve repair scaffold according to any one of claims 1-5, wherein each microchannel has a wall thickness of about 10 µm to about 60 µm.

7. The nerve repair scaffold according to any one of claims 1-6, wherein the scaffold is formed from a biodegradable material.

8. The nerve repair scaffold according to any one of claims 1-7, wherein the scaffold is formed from a mixture comprising poly(ethylene glycol) diacrylate and methacrylated gelatin.

9. The nerve repair scaffold according to claim 8, wherein the scaffold is prepared from about 25% poly(ethylene glycol) diacrylate and about 1-7% methacrylated gelatin.

10. The nerve repair scaffold according to claim 9, wherein the scaffold is formed from a mixture comprising poly(ethylene glycol) diacrylate and methacrylated collagen.

11. The nerve repair scaffold according to any one of claims 1-10, wherein the scaffold further comprises a biofunctional agent.

12. The nerve repair scaffold according to claim 11, wherein the biofunctional agent comprises fibronectin, collagen, laminin, keratin, a growth factor, or a stem cell-promoting factor.

13. The nerve repair scaffold according to any one of claims 1-12, wherein the scaffold comprises an open volume of greater than or equal to about 70%.

14. The nerve repair scaffold according to any one of claims 1-13, wherein the scaffold is 3D printed.

15. The nerve repair scaffold according to claim 7, wherein the biodegradable material is selected from poly(ethylene glycol) diacrylate, methacrylated gelatin, methacrylated collagen, polycaprolactone, and acrylated polycaprolactone, or any combination thereof
